# EUROPÄISCHE PATENTSCHRIFT

(11) **EP 2 832 339 B1**
(45) Veröffentlichungstag und Bekanntmachung des Hinweises auf die Patenterteilung: **01.02.2017**
(21) Anmeldenummer: 14178790.3
(22) Anmeldetag: 28.07.2014
(51) Int. Cl.: A61J 1/14, B65D 85/804

(54) **VORRICHTUNG ZUM HERSTELLEN EINER LÖSUNG, INSBESONDERE IN/AN EINER DIALYSEMASCHINE**
DEVICE FOR PRODUCING A SOLUTION, IN PARTICULAR IN/ON A DIALYSIS MACHINE
DISPOSITIF DE FABRICATION D'UNE SOLUTION, NOTAMMENT DANS OU SUR UN APPAREIL DE DIALYSE

(30) Priorität: 29.07.2013 DE 102013108082
(43) Veröffentlichungstag der Anmeldung: 04.02.2015
(73) Patentinhaber: B. Braun Avitum AG, 34212 Melsungen (DE)
(72) Erfinder: Orszullok, Willy, 58809 Neuenrade (DE)
(74) Vertreter: Winter, Brandl, Fürniss, Hübner, Röss, Kaiser, Polte - Partnerschaft mbB

(56) Entgegenhaltungen:
- WO-A1-2011/161064
- WO-A2-2007/144427
- DE-A1- 10 100 549
- DE-A1- 19 801 107
- DE-A1-102011 017 048

## Beschreibung

Die Erfindung betrifft eine Vorrichtung zum Herstellen einer medizinischen Lösung, insbesondere für den Einsatz in/an einer extrakorporalen Blutbehandlungsmaschine, wie Dialysemaschine, nach dem Oberbegriff von Anspruch 1.

In Dialysemaschinen wird eine Dialysierflüssigkeit durch Auflösen eines in einer Kartusche befindlichen Salzes (Bicarbonat) oder durch Verdünnen eines flüssigen Konzentrats unter Beimischung einer Kartuschen-gespeicherten Flüssigkeit hergestellt. Die Kartusche wird dabei in einem speziellen Kartuschenhalter der Maschine außenseitig gehalten, über den der Kartuscheninhalt bedarfsgerecht abgezapft wird. D.h. dem Kartuschenhalter kommen in der Regel zwei Funktionen zu: das Halten der Kartusche sowie das Abzapfen des Kartuscheninhalts.

Im Stand der Technik sind beispielsweise aus DE 198 52 982 C1 Kartuschenhalter für Dialysemaschinen bekannt, die einen unteren Einspann-/Klemm-Backen und einen oberen Einspann-/Klemm-Backen aufweisen, welche integrierte Anschlüsse für eine Kartusche enthalten. Als Kartuschen werden sogenannte Festbehälter-Kartuschen verwendet mit stabiler Kartuschenwand, die insbesondere als PP-Spritzgussteil hergestellt werden. Die zumeinst zylinderförmigen Kartuschen weisen an ihren gegenüberliegenden Stirnseiten einen Zu- und einen Ablauf auf und sind mit eingelegten oder angeformten Siebelementen ausgestattet zur Verhinderung des Austretens von Pulver oder anderen Schwebstoffen. Hierbei sind die Anschlüsse der Kartusche axial gegenüberliegend angeordnet und durch den steifen/starren Kartuschenkörper auf einem fixen Abstand gehalten. Dadurch kann die Kartusche durch an der Dialysemaschine angeordnete Anschlüsse konnektiert werden, die axial auf die Anschlüsse (ggf. federvorgespannt) aufgedrückt werden. Beutelförmige Behältnisse können aufgrund ihrer mangelnden Form-Stabilität in dem genannten Anschluss-System nicht verwendet werden.

Nachteile der bisherigen Behälter bestehen darin, dass die festen PP-Spritzguss-Kartuschen sehr bruchempfindlich sind und häufig Transportschäden aufweisen. Ferner sind die Siebelemente für das Zurückhalten des Pulvers separate Teile, die besonders befestigt werden müssen. Außerdem neigen feste Kartuschen bei Unterdruck zum Implodieren.

Bei beutelförmigen Behältnissen hingegen, wie sie in der Medizin für andere Zwecke eingesetzt werden (etwa zu Infusionszwecken) gibt es keine Anschlussmöglichkeit an die o.g. Dialysemaschinentypen, und es sind aufwändige Zu- und Ableitungen mit Siebelementen erforderlich.

Die DE 198 01 107 A1 offenbart eine Kartusche zur On-Line-Aufbereitung einer Flüssigkeit. Die Kartusche zur On-Line-Aufbereitung von basischem Bikarbonatdialysekonzentrat besteht aus einem starren Trägerteil und einem flexiblen Beutelteil. Das Trägerteil hat im Wesentlichen eine Rohrform mit einer mittigen Bohrung, in die Osmosewasser eintritt, das aus zahlreichen seitlichen Kanälen weitgehend gleichmäßig verteilt in das das Trägerteil umgebende flexible Beutelteil eintritt. In der Nähe der Austrittsöffnung aus dem Trägerteil sitzt ein Absperrorgan, durch das ebenfalls durch seitliche Kanäle die Konzentratlösung austritt. Nach Entnahme der Kartusche aus dem Kartuschenhalter verschließt das von einer Feder beaufschlagte Absperrorgan die Kartusche.

Die der Erfindung zugrundeliegende Aufgabe ist daher die Schaffung eines Behältnisses, welches in den heute gebräuchlichen extrakorporalen Blutbehandlungsmaschinen, wie Dialysemaschinen mit Kartuschen-Anschlusssystemen verwendet werden kann, die Nachteile im Stand der Technik beseitigt und darüber kostengünstig herzustellen ist.

Diese Aufgabe wird durch die Vorrichtung gemäß Anspruch 1 gelöst. Bevorzugte Ausführungsformen der Erfindung sind Gegenstand der Unteransprüche.

Erfindungsgemäß wird für die Vorrichtung zum Herstellen einer Lösung ein (flexibler) Beutel mit einem innenliegenden Stützrohrelement ausgestattet, wobei das Stützrohrelement einerseits das Anschließen an die Dialysemaschine gestattet, d.h. mit vorzugsweise stirnseitigen Anschlüssen versehen ist und andererseits zugleich das Zu- und Abflusselement für beutelinnere Fluide sowie der Träger (Aufspanneinrichtung) des Beutels ist. Der (flexible) Beutel kann aus zwei einzelnen aufeinandergelegten Folien bestehen, die durch eine rundum verlaufende Schweißnaht verschlossen werden. Durch diese Schweißnaht wird ebenfalls das durch den so entstanden Beutel führende, oben und unten zu Anschlusszwecken aus dem Beutel herausragende Rohr mediendicht (fluiddicht) mit dem Beutel verbunden. Eine weitere Ausführungsform des Beutels kann eine Schlauchfolie als Basis haben.

Die gattungsgemäße Vorrichtung zum Herstellen einer medizinischen Lösung hat:
Ein einen Hohlraum bildendes Behältnis, das zum Aufnehmen von wenigstens einem aufzulösenden Wirkstoff angepasst ist,
wenigstens einen Einlass in den Hohlraum, der zum Zuführen von wenigstens einem Lösungsmittel in den Hohlraum angepasst ist, und
wenigstens einen Auslass aus dem Hohlraum, der zum Ableiten der flüssigen Lösung aus dem wenigstens einen Wirkstoff und dem wenigstens einen Lösungsmittel aus dem Hohlraum angepasst ist,
wobei das Behältnis eine flexible Außenhülle zum Umschließen des Hohlraums und ein inneres, vorzugsweise streben- oder säulenartiges Stützelement zum Aufspannen der flexiblen Außenhülle zwischen zumindest zwei Punkten aufweist, und wobei das Stützelement an seinem ersten (axialen) Ende den wenigstens einen Einlass und an seinem zweiten Ende (axialen) den wenigstens einen Auslass umfasst, und von der flexiblen Außenhülle umschlossen wird.

Die Vorrichtung ist erfindungsgemäß dadurch weiterentwickelt, dass das Stützelement einen rohrförmigen Distanzhalteabschnitt aufweist, mit welchem der Einlass und der Auslass über jeweils ein Rohrbefestigungselement steckbar verbunden sind.

Bevorzugte Ausführungsformen der erfindungsgemäßen Vorrichtung weisen eines oder mehrere der folgenden Merkmale unabhängig oder in Kombination miteinander auf, nach denen
- der Einlass und/oder der Auslass mit einem scheibenförmigen Strömungsleitelement versehen ist/sind, und das Strömungsleitelement mehrere Strömungslenkungseinrichtungen aufweist;
- die Strömungslenkungseinrichtungen von einem Innenumfang des Strömungsleitelements ausgehend geradförmig oder gekrümmt radial zu einem Außenumfang des Strömungsleitelements hin verlaufen
- die flexible Außenhülle eine Schlauchfolie ist, die an ihren beidseitigen Öffnungen mit dem Stützelement dicht verschweißt ist;
- die flexible Außenhülle aus einer ersten und einer zweiten Folie gebildet wird, die an ihrem jeweiligen Rand durch eine Schweißnaht miteinander und mit dem Stützelement verbunden sind, so dass dieses zu Anschlusszwecken oben und unten aus der Außenhülle stutzenförmig herausragt;
- das Stützelement und die Außenhülle durch Durchführungsflansche verbunden ist;
- der Distanzhalteabschnitt des Stützelements rohrförmig ausgebildet ist und unterhalb (stromab) von dem wenigstens einen Einlass und oberhalb (stromauf) von dem wenigstens einen Auslass einen fluiddichten Rohrverschluss oder Pfropfen aufweist;
- der Distanzhalteabschnitt des Stützelements rohrförmig ausgebildet ist und in den Distanzhalteabschnitt unterhalb (stromab) von dem wenigstens einen Einlass und oberhalb (stromauf) von dem wenigstens einen Auslass ein Verschlussstopfen eingepresst ist;
- das Stützelement jeweils wenigstens ein Siebelement nach (stromab zu) dem wenigstens einen Einlass und/oder vor (stromauf zu) dem wenigstens einen Auslass aufweist;
- das Stützelement als Strömungsleitelement jeweils wenigstens ein scheibenförmiges Verteilerelement mit mehreren Verteilerkanälen und/oder mehreren Leitschaufeln als Strömungslenkungseinrichtungen aufweist, das nach (stromab zu) dem wenigstens einen Einlass und/oder vor (stromauf zu) dem wenigstens einen Auslass angeordnet ist, um ein einströmendes Lösungsmittel (Fluid) vorbestimmt (vorzugsweise gleichmäßig) in den Hohlraum zu verteilen und/oder fertige Lösung (9) vorbestimmt in den Auslass zu leiten.

Die erfindungsgemäße Vorrichtung weist u.a. die folgenden Vorteile auf:
Die Vorrichtung ist aufgrund der Tatsache, dass das Stützelement von dem Behältermedium schützend/schlagdämpfend umgeben ist und der Behälter selbst flexibel ist, äußerst bruchstabil ("bruchunempfindlich"), so dass eine erhöhte Transportsicherheit erreicht wird. Die Vorrichtung ist aufgrund der Flexibilität des Behälters (dieser muss keine Einspannkräfte aufnehmen) außerdem unterdruckfest. In der Herstellung ist die Vorrichtung preisgünstig. Ferner ist die Vorrichtung an gängige Dialysemaschine konnektierbar, da das innere Stützelement u. a. die Funktion der aus dem Stand der Technik bekannten starren Behälterwand übernimmt.

Weitere Merkmale und Vorteile der Erfindung ergeben sich aus der folgenden Beschreibung von bevorzugten Ausführungsformen, bei der Bezug genommen wird auf die beigefügten Zeichnungen.
Fig. 1 zeigt ein zu bevorzugten Ausführungsformen der erfindungsgemäßen Vorrichtung hinführendes und nicht Teil der Erfindung bildendes Beispiel im Querschnitt.
Fig. 2 zeigt eine erste bevorzugte Ausführungsform der erfindungsgemäßen Vorrichtung im Querschnitt.
Fig. 3 zeigt eine zweite bevorzugte Ausführungsform der erfindungsgemäßen Vorrichtung im Querschnitt.
Fig. 4 zeigt eine bevorzugte Ausführungsform des erfindungsgemäßen Stützelements in perspektivischer Ansicht.
Fig. 5 zeigt schematisch eine weitere bevorzugte Ausführungsform der erfindungsgemäßen Vorrichtung in perspektivischer Ansicht.
Fig. 6 zeigt schematisch einen Ausschnitt einer weiteren bevorzugten Ausführungsform der erfindungsgemäßen Vorrichtung in perspektivischer Ansicht.
Fig. 7 zeigt schematisch eine bevorzugte Ausführungsform des Endes des erfindungsgemäßen Stützelements im Querschnitt.
Fig. 8 und 9 zeigen schematisch jeweils eine bevorzugte Ausführungsform eines Verteilerelements, das in das erfindungsgemäße Stützelement integriert ist, in Draufsicht.

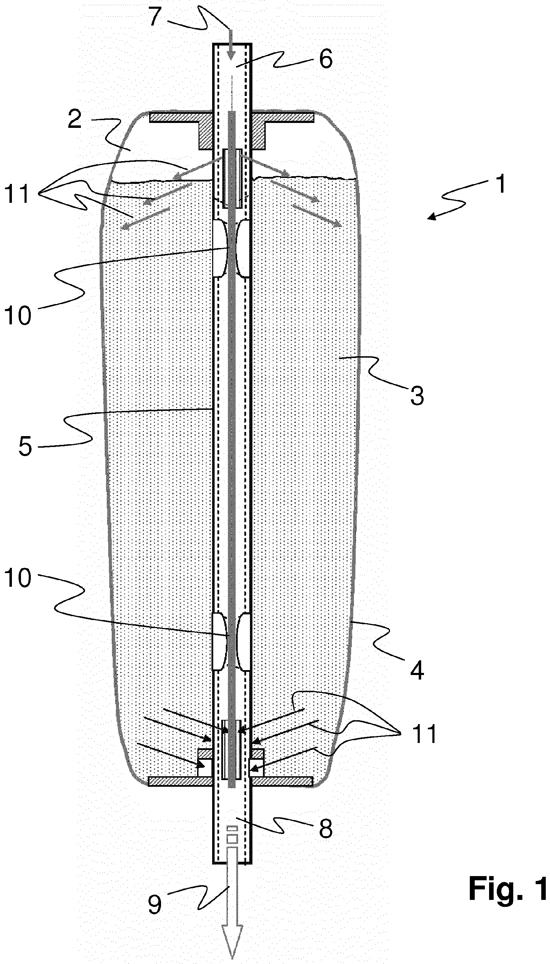

Fig. 1 zeigt ein zu bevorzugten Ausführungsformen der erfindungsgemäßen Vorrichtung hinführendes Beispiel mit einem flexiblen Behältnis oder Beutel 1, der einen Hohlraum 2 umschließt. Der Hohlraum 2 ist ganz oder teilweise mit einem Wirkstoff 3 gefüllt, der in der Regel in körniger Konsistenz oder pulverförmig vorliegt. Die Außenhaut des Beutels 1 ist eine flexible Außenhülle 4, die je nach Ausdehnung ballonförmig oder schlauchförmig den Hohlraum 2 umgibt. Gestützt wird die Außenhülle 4 durch ein inneres (säulenartiges) Stützelement 5, das in der Nähe seines ersten axialen Endes und in der Nähe seines zweiten axialen Endes mit der flexiblen Außenhülle 4 verbunden ist. Das Stützelement 5 ragt mit seinem ersten Ende durch die Außenhülle 4 hindurch, so dass der überstehende Teil des Stützelements 5 als ein Einlass 6 für Lösungsmittel 7 in den Hohlraum 2 der Vorrichtung dient. Das Stützelement 5 ragt außerdem mit seinem zweiten Ende durch die flexible Außenhülle 4 hindurch, so dass dieser überstehende Teil des Stützelements 5 als Auslass 8 für die fertige Lösung 9 dient.

In dem gezeigten Beispiel ist das Stützelement 5 im wesentlichen rohrförmig aufgebaut. Um die Verbindung zwischen dem ersten Ende des Stützelements 5 und dem zweiten Ende des Stützelements 5 innerhalb des Rohrkörpers zu unterbrechen und damit das Lösungsmittel 7 zwangsläufig in dem Hohlraum 2 zu verteilen, ist wenigstens ein Rohrverschluss 10 in dem rohrförmigen Stützelement 5 vorgesehen. Auf diese Art wird sichergestellt, dass sich das Lösungsmittel 7 auf vordefinierten Lösungsmittelpfaden 11 durch den Wirkstoff 3 ausbreiten muss bis in die Nähe des Auslasses 8, wo die Lösung 9 aufgefangen wird und über den Auslass 8 nach außen transportiert wird. Der Rohrverschluss 10 ist vorzugsweise ein heißverprägter Rohrverschluss, der die statische Festigkeit des Stützelements (Rohres) 5 unverändert lässt. Alternativ kann ein Verschluss-Stopfen eingepresst werden.

Einlass 6 und Auslass 8 sind auf beiden axialen Seiten des Behälters 1 gleich und daher beliebig einbaubar. Für das Einleiten der Flüssigkeit über den Einlass in das Behälter-innere Medium sowie für das Ausleiten der Lösung zum Auslass sind Verteiler- bzw. Sammelöffnungen im Stützelement 5 vorgesehen, wobei die Sammelöffnungen für die fertige Lösung 9 kurz oberhalb (stromauf) des eigentlichen Auslasses 8 schlitzförmig im Stützelement 5 angelegt sein können und z.B. durch Laser-Schneidprozesse oder Feinstanzen erzeugt werden.

Das Befüllen des erfindungsgemäßen Beutels 1 mit pulverförmigen Stoffen erfolgt vorzugsweise durch eine Öffnung oben (im Bereich des Einlasses 6) im Beutel. Zum Befüllen wird das innere Stützelement 5 beiseite gedrückt, und der Wirkstoff wird trocken eingefüllt. Anschließend wird der Beutel 1 dicht an der Einfüllöffnung 23 verschweißt. Alternativ kann die Öffnung oben seitlich vom Einlass 6 angeordnet sein und verschweißt werden oder einen separaten Verschluss aufweisen.

Ein solches, in Fig. 1 gezeigtes beutelförmiges Behältnis 1 für pulverförmige Wirkstoffe (z.B. Bicarbonat) mit zentralem Stütz- und Anschlussrohr ist besonders geeignet für die Verwendung in Dialysemaschinen, bei denen das Behältnis 1 zwischen zwei (nicht gezeigten) festgelegten Zu- und Ablaufanschlüssen eingespannt wird. Der flexible Beutel 1 mit dem axialstabilen Stützelement 5 als Zentralelement vereinigt alle erforderlichen Funktionen in sich:
- Anschlussmöglichkeit an Dialysemaschinen führender Hersteller,
- Zu- und Ablauffunktion von Lösungsmittel 7 bzw. fertiger Lösung 9,
- Verhindern einer direkten Verbindung von Zu- und Ablauf, so dass eine homogene Durchmischung gewährleistet ist,
- Siebfunktion gegen das Austreten von Pulver durch entsprechende, weiter unten erläuterte Maßnahmen,
- Verbindungsbereiche des Stützelements 5 zum vereinfachten Verschweißen mit dem Beutel 1,
- Sicherheit gegen Implosion bei Unterdruck.

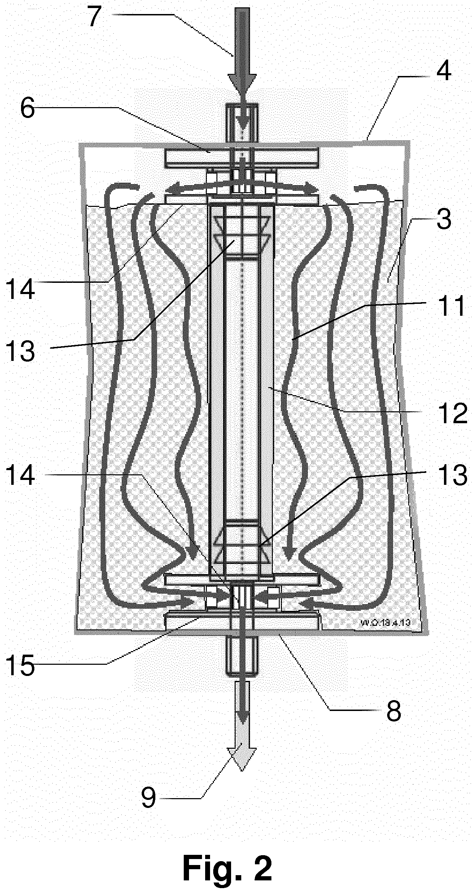

Fig. 2 zeigt eine erste Ausführungsform des Beutels 1 mit der flexiblen Außenhülle 4, in der sich der pulverförmige Wirkstoff 3 befindet. Über ein oberes, am Stützelement angeordnetes Träger- und Verteilerelement als Einlass 6 gelangt der Flüssigkeitszustrom des Lösungsmittels 7 in vorbestimmter Verteilungs- oder Versprühweise zu dem Wirkstoff 3. Das Stützelement 5 setzt sich in dieser Ausführungsform aus einem Rohr 12 und zwei Endstücken zusammen, die über jeweils einen Rohrbefestigungsdübel 13 mit dem Rohr 12 verbunden sind. Mit anderen Worten, die Zu- und Ablaufanschlüsse 6 und 8 sind auf das (Träger- und Distanzhalte-) Rohr 12 aufgesteckt.

Die Anschlussstücke 6 bzw. 8, insbesondere der Zulaufbereich des Einlasses 6, sind direkt hinter (stromab) den Verteiler-/Sammelöffnungen in dem Stützelement 5 mit einer scheibenförmigen Verteilerplatte 14 (Strömungsleitelement) ausgestattet, die ein direktes Herunterlaufen der zugeführten Flüssigkeit am Trägerrohr verhindert und so einer kernförmigen Ausspülung des pulverförmigen Wirkstoffes um die Mittelachse vorbeugt. Gleichzeitig wird eine gute Vermischung von Flüssigkeit und Feststoffen gefördert. Dies ist durch die Lösungsmittelpfade 11 in Fig. 2 angedeutet.

Die Anschlussstücke 6 bzw. 8 können darüber hinaus insbesondere mit Gitterstäben oder einer siebartigen Oberfläche versehen sein, so dass körnige Wirkstoffe zurückgehalten werden. Dieses Siebelement 15 befindet sich vorzugsweise unmittelbar vor /stromauf zu) dem Auslass 8.

Die Außenhülle 4 des Beutels 1 ist mit dem Trägerelement 5 und den Anschluss-Stücken 6 bzw. 8 fluiddicht verschweißt. Weitere Ausführungsformen sehen am Rohr 12 durch Spritzgießen oder Schweißen angeformte flanschartige Teller vor, auf die die gelochte Außenhülle 4 aufgeschweißt/aufgeklebt werden kann. Weiterhin kann die Verbindung zwischen Stützelement 5 und Beutel 1 durch schiffchenförmige Ansätze am Rohr 12 erfolgen, die mit der Außenhülle 4 verschweißt werden.

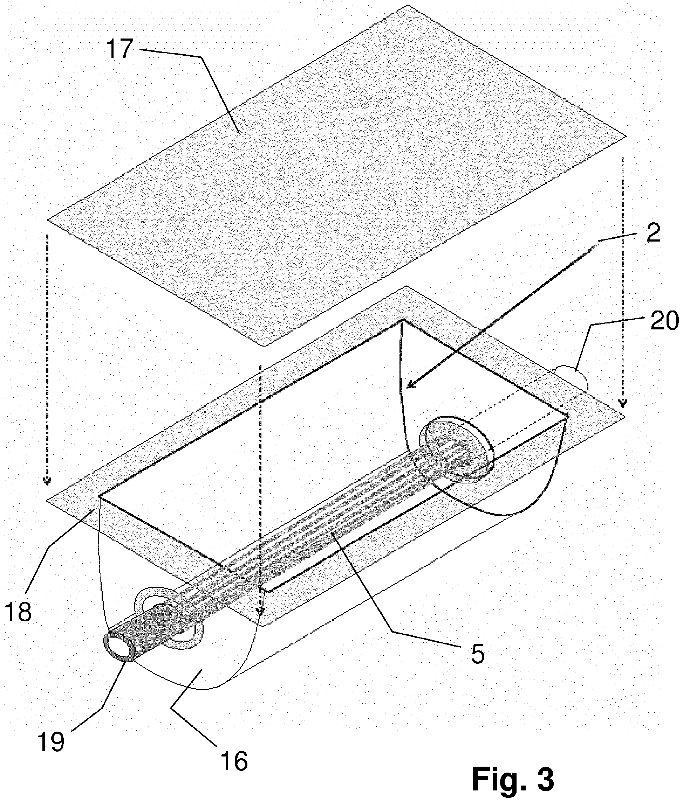

Die Außenhülle 4 des Beutels 1 in dem Beispiel nach Fig. 1 und in der Ausführungsform nach 2 ist einstückig schlauchförmig dargestellt. Anstelle einer schlauchförmigen Außenhülle 4 kann aber auch eine aus zwei Folien zusammengesetzte Außenhülle 4 für den Beutel 1 verwendet werden. Eine derartige Außenhülle ist in Fig. 3 gezeigt. Die Außenhülle 4 setzt sich aus einer ersten Folie 16 und einer zweiten Folie 17 zusammen. In der dargestellten Ausführungsform nimmt die erste Folie 16 in maximal ausgedehntem Zustand eine Trogform an. Durch den Trog verläuft das Stützelement 5, das an den zwei Stirnseiten des Trogs mit seinem ersten Ende 19 und seinem zweiten Ende 20 durch die erste Folie 16 nach außen tritt. Die erste Folie 16 bildet zusammen mit der zweiten Folie 17 einen abgeschlossenen Hohlraum 2. Die erste Folie 16 und die zweite Folie 17 werden über eine Schweißnaht 18 miteinander verbunden.

Die in Fig. 3 gezeigte Ausführungsform mit der trogförmigen ersten Folie 16 bietet den Vorteil einer großen Einfüllöffnung 23 für den aufzulösenden Wirkstoff im Beutel 1.

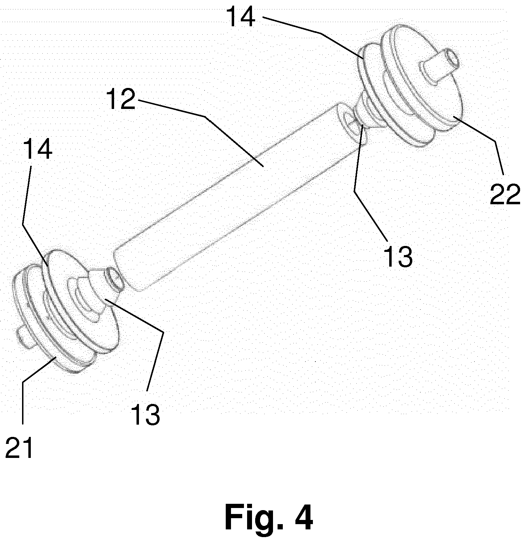

In Fig. 4 ist eine Ausführungsform des erfindungsgemäßen Stützelements 5 in perspektivischer Darstellung gezeigt. In dieser Ausführungsform setzt sich das Stützelement 5 aus einem Rohr 12 und einem ersten Durchführungsflansch 21 sowie einem zweiten Durchführungsflansch 22 zusammen. Die beiden Durchführungsflansche 21, 22 sind im Wesentlichen gleichartig. Sie verfügen über den erforderlichen rohrförmigen Anschluss an eine Dialysemaschine, der als Einlass oder Auslass wie in Fig. 1 gezeigt dient. Unterhalb bzw. oberhalb des Anschlusses sind ein oder mehrere siebförmige Ein- bzw. Auslässe (Verteiler-/Sammelöffnungen) vorgesehen. Die Durchführungsflansche 21, 22 können rund oder schiffchenförmig sein und stellen den Verbindungsbereich mit dem Beutel dar. An dem der Anschlussöffnung gegenüberliegenden Ende sind sie jeweils mit einem Rohrbefestigungsdübel (Blindstopfen) 13 versehen. Der Rohrbefestigungsdübel 13 wird eine Rohraufnahme in dem Rohr 12 (Rohrinnenraum) axial eingedrückt (eingepresst) und so mit dem Rohr 12 zu dem erfindungsgemäßen Stützelement 5 verbunden. Der Rohrbefestigungsdübel 13 ist vorzugsweise nicht durchgängig, so dass gleichzeitig ein Rohrverschluss 10 gebildet wird, der verhindert, dass Flüssigkeit durch das Rohr 12 fließt.

Durch Aufstecken der Durchführungsflansche 21, 22 auf das jeweilige Ende des z.B. extrudierten Rohres 12 wird so das tragende Stützelement 5 des Beutels 1 gebildet. Das Stützrohr 12 wird dann durch Schweißen mit dem Beutel 1 verbunden.

Die Durchführungsflansche 21, 22 sind insbesondere beide vorzugsweise mit einer Flüssigkeitsverteilerplatte 14 ausgestattet, wie sie auch in Fig. 2 gezeigt ist.

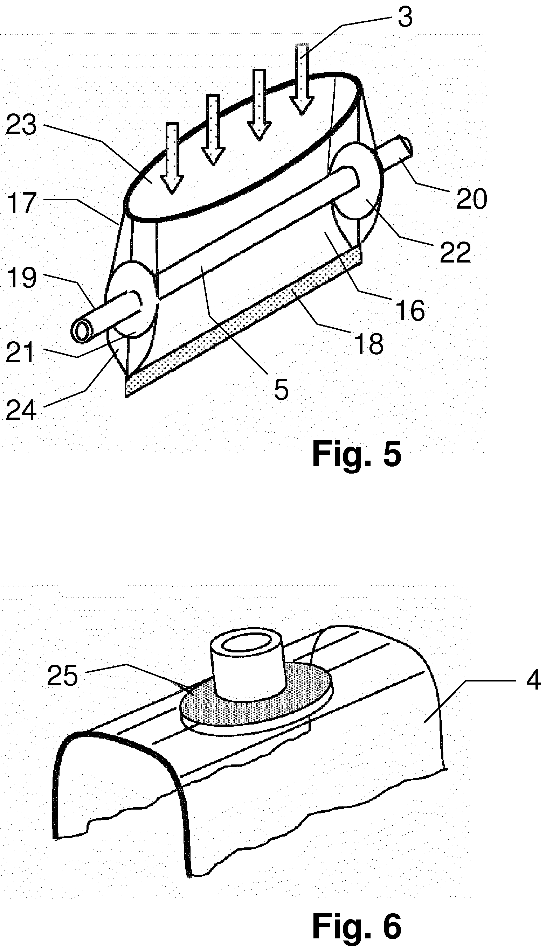

Die Befüllung des Beutels 1 kann über eine offene Seiten- oder auch Teil-Naht erfolgen, die nach dem Befüllvorgang verschlossen wird. Dies wird im folgenden anhand von Fig. 5 erläutert. In Fig. 5 ist ein Beutel 1 mit einer Außenhülle 4 aus zwei Folien 16 und 17 gezeigt. Die Folien 16, 17 sind an ihrer unteren Seite über eine Schweißnaht 18 miteinander verbunden. Die gegenüberliegende obere Schweißnaht ist noch nicht geschlossen, so dass sich eine Einfüllöffnung 23 für den einzufüllenden Wirkstoff 3 ergibt. Diese Einfüllöffnung 23 kann in Abhängigkeit von der Länge der Schweißnaht 18 so breit gewählt werden, dass das Einfüllen leicht durchführbar ist und andererseits bereits beim Einfüllen eine ausreichende Stabilität des Beutels 1 gewährleistet ist.

An ihrer Seite sind die beiden Folien 16, 17 über ein Seitenteil 24 miteinander verbunden. Das Seitenteil 24 ist eine balgförmige Folie zwischen den Folien 16, 17. Mit dem Seitenteil 24 ist das Stützelement 5 verbunden, und das Stützelement 5 durchstößt mit seinen Endabschnitten 19 und 20 jeweils einen Seitenteil 24, wobei der Übergang zwischen Stützelement 5 und Seitenteil 24 durch den jeweiligen Flansch 21, 22 abgedichtet wird.

Die Durchführung des Stützelements 5 durch eine Folie ist nicht auf im wesentlichen ebene oder balgförmige Folien beschränkt. Eine alternative Ausführungsform zeigt Fig. 6. Aufgrund eines stabilen Durchführungsflansches 25 kann das Stützelement 5 auch durch eine stärker gekrümmte Außenhülle 4 hindurch geführt werden.

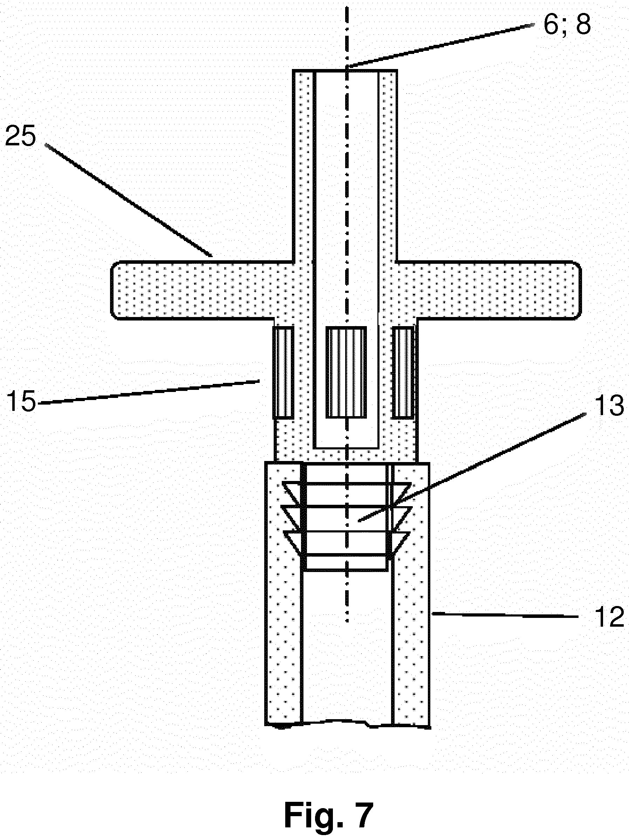

Der Aufbau des Durchführungsflansches 25 ist nochmals in Fig. 7 dargestellt. Der Flansch 25 dient mit seinem integrierten Flüssigkeitskanal als Einlass 6 bzw. Auslass 8. Über einen Dübel 13 ist der Durchführungsflansch 25 mit dem Rohr 12 des erfindungsgemäßen Stützelements 5 verbunden, wobei keine Flüssigkeit von dem Flansch 25 in das Rohr 12 gelangt. Stattdessen ist unterhalb des Flansches ein Siebelement 15 angeordnet, durch das die zugeführte Flüssigkeit in den Beutel 1 strömt und damit den darin enthaltenen Wirkstoff benetzt. Das Siebelement 15 kann insbesondere in Zusammenhang mit der oben bereits beschriebenen Flüssigkeitsverteilerplatte 14 als Zulaufverteiler des Lösungsmittels dienen.

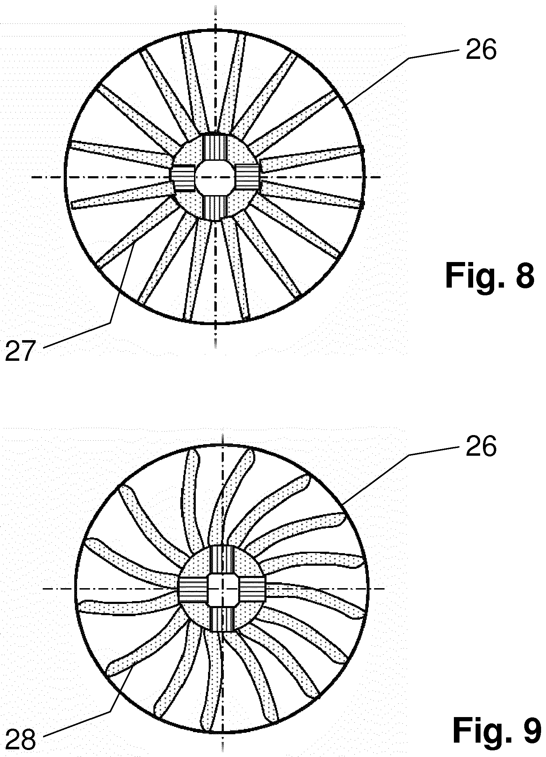

Ein solches Verteilerelement 26 mit Siebfunktion ist in Fig. 8 und 9 in Draufsicht gezeigt. Dabei wird davon ausgegangen, dass unterhalb des Flüssigkeitszulaufes wie in Fig. 2 und 4 gezeigt eine Verteilerplatte 14 angeformt ist. Diese Verteilerplatte dient der Verteilung des Zustromes auf eine größere Pulveroberfläche und verhindert gleichzeitig das vorzeitige Ausspülen des pulverförmigen Wirkstoffes entlang der Mittelachse, welches zu einer verminderten Konzentration der zu bildenden Lösung führen kann. Ein Verteilerelement 26 ist zur Strömungslenkung mit Verteilerkanälen 27 oder Leitschaufeln 28 in gerader oder auch gekrümmter Form ausgestattet. Damit lässt sich dann eine optimale Benetzung des im Beutel 1 vorhandenen Wirkstoffes erreichen.

Die erfindungsgemäße Vorrichtung ist nicht auf die dargestellten Ausführungsformen beschränkt. Insbesondere kann bei dem Folienbehälter für pulverförmige Wirkstoffe für den geschweißten Beutel eine Mehrschicht- oder auch Monofolie als Material eingesetzt werden. Es eignet sich z.B. eine Folie aus den Schichten PE/PA/PE, die mit einer Gesamtstärke von 200 Micron koextrudiert ist, oder eine Folie aus den Schichten PP/PA/PE, die mit einer Gesamtstärke von 150 Micron koextrudiert ist, oder eine Monofolie aus PP mit einer Schichtstärke von bis zu 900 Micron. Die verwendeten Folien-Wandstärken können zwischen 150 Mircon und 900 Micron liegen. Alle genannten Folien sind auch mit entsprechenden Wandstärken für den Einsatz als Tiefziehfolie verwendbar. Diese Folien zeichnen sich durch hohe Reißfestigkeiten aus, was wiederum für eine Unterdruckfestigkeit und Unterdruckdichtigkeit des daraus hergestellten Beutels 1 bei den Betriebsarten des Unterdruckleersaugens des Behältnisses sehr vorteilhaft ist, da die Gefahr des Implodierens, wie sie bei starren Kartuschenkörpern gegeben ist, stark verringert werden kann. Innendruckbelastungen des Beutels von bis zu 3 bar sind mit den vorgenannten Materialien durchaus realisierbar.

Außerdem können auf die Zu- und Ablauf-Anschlussstutzen nach dem Befüllen des Behältnisses 1 mit Wirkstoff 3 zwecks hygienischer Versiegelung und gg. auch als Originalitätsverschluss jeweils eine Abreißfolie mit Handhabungslasche aufgeschweißt werden, welche vor der Benutzung durch Abziehen entfernt werden. Ebenso sind aufgesteckte und mit den Behälter-Anschlüssen verriegelte Abbrech- oder Abdrehkappen als Originalitätsverschluss denkbar.

Die Erfindung betrifft zusammenfassend eine Vorrichtung zum Herstellen einer Lösung, die umfasst: Ein einen Hohlraum (2) bildendes Behältnis (1) zum Aufnehmen von wenigstens einem aufzulösenden Wirkstoff (3), wenigstens einen Einlass (6) in den Hohlraum (2)zum Zuführen von wenigstens einem Lösungsmittel (7) in den Hohlraum (2) und wenigstens einen Auslass (8) aus dem Hohlraum (2) zum Ableiten der flüssigen Lösung (9) aus dem wenigstens einen Wirkstoff (3) und dem wenigstens einen Lösungsmittel (7) aus dem Hohlraum (2). Zur Schaffung einer axial starren Kartusche, welche in den heute gebräuchlichen Dialysemaschinen mit Kartuschen-Anschlusssystemen verwendet werden kann und kostengünstig herzustellen ist, wird erfindungsgemäß vorgeschlagen, dass der Hohlraum (2) bzw. das Behältnis (1) aufweist: eine flexible Außenhülle (4) zum Umschließen des Hohlraums (2) und ein säulenartiges, inneres Stützelement (5) zum Aufspannen der flexiblen Außenhülle (4) zwischen wenigstens zwei Punkten, wobei das Stützelement (5) an seinem ersten Ende (19) den wenigstens einen Einlass (6) und an seinem zweiten Ende (20) den wenigstens einen Auslass (8) umfasst und von der flexiblen Außenhülle (4) umschlossen wird.

### Bezugszeichen

- 1: Behälter, Beutel, Behältnis
- 2: Hohlraum
- 3: Wirkstoff
- 4: flexible Außenhülle, Schlauchfolie
- 5: Stützelement
- 6: Einlass
- 7: Lösungsmittel
- 8: Auslass
- 9: Lösung
- 10: Rohrverschluss
- 11: Lösungsmittelpfade
- 12: Rohr
- 13: Rohrbefestigungsdübel
- 14: Flüssigkeitsverteilerplatte
- 15: Siebelement
- 16: erste Folie
- 17: zweite Folie
- 18: Schweißnaht
- 19: erstes Ende von Stützelement
- 20: zweites Ende von Stützelement
- 21: erster Durchführungsflansch
- 22: zweiter Durchführungsflansch
- 23: Einfüllöffnung
- 24: Seitenteil
- 25: Durchführungsflansch
- 26: Verteilerelement
- 27: Verteilerkanäle
- 28: Leitschaufeln

## Patentansprüche

1. Vorrichtung zum Herstellen einer medizinischen Lösung (9), die folgende Teile hat:
ein einen Hohlraum (2) ausbildendes Behältnis (1) zum Aufnehmen von wenigstens einem aufzulösenden Wirkstoff (3),
wenigstens einen Einlass (6) in den Hohlraum (2) zum Zuführen von wenigstens einem Lösungsmittel (7) in den Hohlraum (2), und
wenigstens einen Auslass (8) aus dem Hohlraum (2) zum Ableiten der flüssigen Lösung (9) aus dem wenigstens einen Wirkstoff (3) und dem wenigstens einen Lösungsmittel (7) aus dem Hohlraum (2),
wobei das Behältnis (1) eine flexible Außenhülle (4) zum Umschließen des Hohlraums (2) und ein inneres, säulenartiges Stützelement (5) zum Aufspannen der flexiblen Außenhülle (4) zwischen zumindest zwei Punkten aufweist, und
wobei das Stützelement (5) an seinem ersten Ende (19) den wenigstens einen Einlass (6), an seinem zweiten Ende (20) den wenigstens einen Auslass (8) aufweist, und von der flexiblen Außenhülle (4) umschlossen wird,
**dadurch gekennzeichnet, dass**
das Stützelement (5) einen rohrförmigen Distanzhalteabschnitt (12) aufweist, mit welchem der Einlass (6) und der Auslass (8) über jeweils ein Rohrbefestigungselement (13) steckbar verbunden sind.

2. Vorrichtung nach Anspruch 1, bei der der Einlass (6) und/oder der Auslass (8) mit einem scheibenförmigen Strömungsleitelement (14) versehen ist/sind, und das Strömungsleitelement (14) mehrere Strömungslenkungseinrichtungen (27, 28) aufweist.

3. Vorrichtung nach Anspruch 2, bei der die Strömungslenkungseinrichtungen (27, 28) von einem Innenumfang des Strömungsleitelements (14) ausgehend geradförmig oder gekrümmt radial zu einem Außenumfang des Strömungsleitelements (14) hin verlaufen.

4. Vorrichtung nach einem der Ansprüche 1 bis 3, bei der die flexible Außenhülle (4) eine Schlauchfolie ist, die an ihren beidseitigen Öffnungen mit dem Stützelement (5) dicht verschweißt ist.

5. Vorrichtung nach einem der Ansprüche 1 bis 3, bei der die flexible Außenhülle (4) aus einer ersten (16) und einer zweiten Folie (17) gebildet wird, die an ihrem jeweiligen Rand durch eine Schweißnaht (18) miteinander und mit dem Stützelement (5) verbunden sind, so dass dieses zu Anschlusszwecken oben und unten aus der Außenhülle (4) stutzenförmig herausragt.

6. Vorrichtung nach einem der vorangehenden Ansprüche, bei der das Stützelement (5) und die Außenhülle (4) durch Durchführungsflansche (21, 22; 25) verbunden ist.

7. Vorrichtung nach einem der vorangehenden Ansprüche, bei der der Distanzhalteabschnitt (12) des Stützelements (5) rohrförmig ausgebildet ist und unterhalb von dem wenigstens einen Einlass (6) und oberhalb von dem wenigstens einen Auslass (8) einen fluiddichten Rohrverschluss oder Pfropfen (10) aufweist.

8. Vorrichtung nach einem der Ansprüche 1 bis 6, bei der der Distanzhalteabschnitt (12) des Stützelements (5) rohrförmig ausgebildet ist und in den Distanzhalteabschnitt (12) unterhalb von dem wenigstens einen Einlass (6) und oberhalb von dem wenigstens einen Auslass (8) ein Verschlussstopfen eingepresst ist.

9. Vorrichtung nach einem der vorangehenden Ansprüche, bei der das Stützelement (5) jeweils wenigstens ein Siebelement (15) nach dem wenigstens einen Einlass (6) und/oder vor dem wenigstens einen Auslass (8) aufweist.

10. Vorrichtung nach einem der vorangehenden Ansprüche, bei der das Stützelement (5) als Strömungsleitelement (14) jeweils wenigstens ein scheibenförmiges Verteilerelement (26) mit mehreren Verteilerkanälen (27) und/oder mehreren Leitschaufeln (28) als Strömungslenkungseinrichtungen (27, 28) aufweist, das nach dem wenigstens einen Einlass (6) und/oder vor dem wenigstens einen Auslass (8) angeordnet ist, um einströmendes Lösungsmittel (7) vorbestimmt in den Hohlraum (2) zu verteilen und/oder fertige Lösung (9) vorbestimmt in den Auslass (8) zu leiten.

## Claims

1. A device for preparing a medical solution (9), comprising the following parts:
a receptacle (1) defining a cavity (2) and intended for receiving at least one active substance (3) to be dissolved,
at least one inlet (6) leading into the cavity (2) and intended for feeding at least one solvent (7) into the cavity (2), and
at least one outlet (8) leading out of the cavity (2) and intended for discharging the liquid solution (9) consisting of the at least one active substance (3) and the at least one solvent (7) from the cavity (2),
wherein the receptacle (1) comprises a flexible outer shell (4) for enclosing the cavity (2) and an inner, columnar supporting element (5) for stretching the flexible outer shell (4) between at least two points, and
the supporting element (5) comprising the at least one inlet (6) at its first end (19) and the at least one outlet (8) at its second end (20), and being enclosed by the flexible outer shell (4),
**characterized in that**
the supporting element (5) comprises a tube-shaped spacer portion (12) with which the inlet (6) and the outlet (8) are each attachably connected via a respective tube fastening stud (13).

2. The device according to claim 1, in which the inlet (6) and/or the outlet (8) is/are equipped with a disc-shaped flow guiding element (14), and the flow guiding element (14) comprises several flow guiding devices (27, 28).

3. The device according to claim 2, in which the flow guiding devices (27, 28), starting from an inner circumference of the flow guiding element (14), run in a straight or also curved shape toward the outer circumference of the flow guiding element (14).

4. The device according to any one of claims 1 to 3, in which the flexible outer shell (4) is a tubular foil which has its both openings tightly welded to the supporting element (5).

5. The device according to any one of claims 1 to 3, in which the flexible outer shell (4) is formed from a first foil (16) and a second foil (17) which each have their respective edges connected to each other and to the supporting element (5) by a weld seam (18), so that the supporting element protrudes for connecting purposes from the outer shell (4) at the top and at the bottom in the manner of a connecting piece.

6. The device according to any of the preceding claims, in which the supporting element (5) and the outer shell (4) are connected by feed-through flanges (21, 22; 25).

7. The device according to any of the preceding claims, in which the spacer portion (12) of the supporting element (5) has a tube shape and comprises a fluid-tight tube closure or plug (10) underneath the at least one inlet (6) and above the at least one outlet (8).

8. The device according to any of the claims 1 to 6, in which the spacer portion (12) of the supporting element (5) has a tube shape and wherein a closure plug is pressed into the spacer portion (12) underneath the at least one inlet (6) and above the at least one outlet (8).

9. The device according to any of the preceding claims, in which the supporting element (5) comprises at least one sieve element (15) behind the at least one inlet (6) and/or in front of the at least one outlet (8).

10. The device according to any of the preceding claims, in which the supporting element (5) as flow guiding element (14) comprises at least one respective disc-shaped distribution element (26) equipped with several distribution channels (27) and/or several guide vanes (28) as flow guiding devices (27, 28), wherein the one disc-shaped distribution element is arranged behind the at least one inlet (6) and/or in front of the at least one outlet (8), in order to distribute inflowing solvent (7) in the cavity (2) in a predetermined manner and/or to convey the prepared solution (9) into the outlet (8) in a predetermined manner.

## Revendications

1. Dispositif de fabrication d'une solution médicale (9), qui a les parties suivantes :
un contenant (1) formant une cavité (2) pour la réception d'au moins un principe actif à dissoudre (3),
au moins une entrée (6) dans la cavité (2) pour l'acheminement d'au moins un solvant (7) dans la cavité (2), et
au moins une sortie (8) de la cavité (2) pour l'évacuation de la solution liquide (9) d'au moins un principe actif (3) et d'au moins un solvant (7) de la cavité (2),
dans lequel le contenant (1) présente une enveloppe extérieure flexible (4) pour l'entourage de la cavité (2) et un élément d'appui intérieur de type colonne (5), pour le serrage de l'enveloppe extérieure flexible (4) entre au moins deux points, et
dans lequel l'élément d'appui (5) à sa première extrémité (19) présente l'au moins une entrée (6), à sa deuxième extrémité (20), présente l'au moins une sortie (8) et est entouré de l'enveloppe flexible (4),
**caractérisé en ce que**
l'élément d'appui (5) présente un segment de maintien de distance tubulaire (12) avec lequel l'entrée (6) et la sortie (8) sont reliées de façon enfichable par respectivement un élément de fixation tubulaire (13).

2. Dispositif selon la revendication 1, dans lequel l'entrée (6) et/ou la sortie (8) est/sont dotée(s) d'un élément conducteur de courant sous forme de disque (14), et l'élément conducteur de courant (14) présente plusieurs systèmes de déviation d'écoulement (27, 28).

3. Dispositif selon la revendication 2, dans lequel les systèmes de déviation de l'écoulement (27, 28) s'étendent à partir d'un pourtour intérieur de l'élément conducteur de courant (14) en une forme linéaire ou radialement courbée jusqu'à un pourtour extérieur de l'élément conducteur de courant (14).

4. Dispositif selon l'une des revendications 1 à 3, dans lequel l'enveloppe extérieure flexible (4) est un film tubulaire qui est hermétiquement soudé à ses ouvertures bilatérales avec l'élément d'appui (5).

5. Dispositif selon l'une des revendications 1 à 3, dans lequel l'enveloppe extérieure flexible (4) est formée d'un premier (16) et d'un deuxième film (17) qui sont reliés l'un à l'autre sur leur bord respectif par une soudure (18) et avec l'élément d'appui (5) de sorte que celui-ci dépasse vers le haut et vers le bas de l'enveloppe extérieure (4) sous forme d'embout, à des fins de raccordement.

6. Dispositif selon l'une des revendications précédentes, dans lequel l'élément d'appui (5) et l'enveloppe extérieure (4) sont reliés par une bride traversante (21, 22 ; 25).

7. Dispositif selon l'une des revendications précédentes, dans lequel le segment de maintien de distance (12) de l'élément d'appui (5) est conçu de façon tubulaire et présente, en-dessous de l'au moins une entrée (6) et au-dessus de l'au moins une sortie (8), un raccordement tubulaire étanche aux fluides ou un bouchon (10).

8. Dispositif selon l'une des revendications 1 à 6, dans lequel le segment de maintien de distance (12) de l'élément d'appui (5) est conçu de façon tubulaire et dans le segment de maintien de distance (12), en-dessous de l'au moins une entrée (6) et au-dessus de l'au moins une sortie (8), est inséré un bouchon d'obturation.

9. Dispositif selon l'une des revendications précédentes, dans lequel l'élément d'appui (5) présente au moins un élément de tamisage (15) après l'au moins une entrée (6) et/ou avant l'au moins une sortie (8).

10. Dispositif selon l'une des revendications précédentes, dans lequel l'élément d'appui (5) présente comme élément conducteur de courant (14) respectivement au moins un élément de répartition en forme de disque (26) comportant plusieurs canaux de répartition (27) et/ou plusieurs ailettes directrices (28) en tant que systèmes de déviation d'écoulement (27, 28), qui est disposé après l'au moins une entrée (6) et/ou avant l'au moins une sortie (8) pour répartir un solvant entrant (7) de façon prédéterminée dans la cavité (2) et/ou pour conduire une solution terminée (9) de façon prédéterminée dans la sortie (8).
